# EUROPÄISCHE PATENTSCHRIFT

(11) **EP 1 768 953 B1**
(45) Veröffentlichungstag und Bekanntmachung des Hinweises auf die Patenterteilung: **14.05.2008**
(21) Anmeldenummer: 05753206.1
(22) Anmeldetag: 22.06.2005
(51) Int. Cl.: C07C 231/24

(54) **VERFAHREN ZUR REINIGUNG VON DIMETHYLACETAMID (DMAC)**
METHOD FOR THE PURIFICATION OF DIMETHYLACETAMID (DMAC)
PROCEDE DE PURIFICATION DE DIMETHYLACETAMIDE (DMAC)

(30) Priorität: 24.06.2004 DE 102004030616
(43) Veröffentlichungstag der Anmeldung: 04.04.2007
(73) Patentinhaber: BASF SE, 67056 Ludwigshafen (DE)
(72) Erfinder: PESCHEL, Werner, 67251 Freinsheim (DE); KIRCHNER, Tanja, 55283 Nierstein (DE); RUST, Harald, 67435 Neustadt (DE)
(74) Vertreter: Isenbruck, Günter
(86) Internationale Anmeldenummer: PCT/EP2005/006732
(87) Internationale Veröffentlichungsnummer: WO 2006/000392

(56) Entgegenhaltungen:
- WO-A-20/04002926
- WO-A-20/04087639

## Beschreibung

Die Erfindung betrifft ein Verfahren zur destillativen Reinigung von Roh-Dimethylacetamid. Für Dimethylacetamid wird im Folgenden die abgekürzte Bezeichnung DMAc verwendet.

DMAc wird überwiegend als Lösungsmittel eingesetzt, beispielsweise als Lösungsmittel zum Lösungsspinnen vorn elastischen Polyurethan-Blockcopolymeren, die unter dem Markennamen Spandex^{®} oder Lycra^{®} bekannt sind, wie auch zur Herstellung von Hohlfasern.

Um beim Lösungsspinnen qualitativ hochwertige Fasern erhalten zu können, werden an das hierbei eingesetzte DMAc die nachfolgend aufgeführten Spezifikationenanforderungen gestellt: Wassergehalt < 100 ppm, pH-Wert zwischen 6,5 und 7 und spezifische elektrische Leitfähigkeit < 0,6 µS/cm,oder auch < als 0,2 µS/cm. Die elektrische Leitfähigkeit des Rein-DMAc wird im Wesentlichen durch seinen Gehalt an Verunreinigungen, wie Säuren, insbesondere Essigsäure und Salzen, insbesondere den Aminsalzen der Essigsäure, bewirkt. Die angegebenen Spezifikationen des Rein-DMAc bezüglich pH-Wert und elektrischer Leitfähigkeit entsprechen einem Gehalt an Essigsäure von weniger als 50 Gew.-ppm.

Ein DMAc, das diese Spezifikationsanforderungen erfüllt, wird im Folgenden als Rein-DMAc bezeichnet.

Demgegenüber wird als Roh-DMAc ein Gemisch, enthaltend DMAc, bezeichnet, das die oben definierten Spezifikationsanforderungen nicht erfüllt. Roh-DMAc enthält als Hauptkomponenten neben DMAc Amine, Essigsäure und Wasser. Hierbei ist der Gesamtgehalt an Säuren, überwiegend Essigsäure, daneben auch Spuren an Ameisensäure sowie an höheren Carbonsäuren, von erfindungsgemäß zu reinigendem Roh-DMAc nicht größer als 20 Gew.-%, bezogen auf DMAc.

Als wasserhaltiges Roh-DMAc wird vorliegend ein Roh-DMAc mit einem Wassergehalt zwischen 1 und 99 Gew.-%, oder auch zwischen 2 und 99 Gew.-%, auch zwischen 80 und 98 Gew.-%, insbesondere zwischen 95 und 98 Gew.-%, oder auch zwischen 1 und 6 Gew.-% verstanden.

Bekannte Anlagen zur destillativen Reinigung von DMAc werden unter Vakuum betrieben, um Zersetzungsreaktionen des DMAc wirksam zu unterdrücken und dadurch die Einhaltung der Spezifikationsanforderungen zu gewährleisten.

Es wurde jedoch gefunden, dass in Destillationsanlagen, die wie bislang üblich unter Vakuum betrieben wurden, die Spezifikationsanforderungen häufig nicht erfüllt werden können.

WO 2004/087 639 offenbart die Reinigung von DMAc.

Die FR-PS 1,406,279 beschreibt ein Verfahren zur Herstellung von DMAc durch Umsetzung von Essigsäure mit Dimethylamin und destillative Auftrennung des Reaktionsgemisches in einer Zweikolonnen-Anordnung, wobei die erste Kolonne bei Atmosphärendruck oder geringfügig erhöhtem Druck, einer Sumpftemperatur zwischen 165 und 170°C, einer Kopftemperatur zwischen 95 und 105°C und einer Temperatur zwischen 100 und 200°C im Bereich des Kolonnen-Feeds, der etwa in der Mitte der Kolonne angeordnet ist, betrieben wird. Aus der ersten Kolonne wird ein Kopfstrom, enthaltend Dimethylamin und Wasser abgezogen und ein gasförmiger oder flüssiger Sumpfstrom, enthaltend DMAc, Essigsäure sowie Monomethylacetamid. Dieser Sumpfstrom wird einer zweiten Kolonne, etwa im mittleren Bereich derselben, zugeführt und in einen Kopfstrom, enthaltend gereinigtes DMAc und einen Sumpfstrom, enthaltend das ternäre Azeotrop von Essigsäure, DMAc und Monomethylacetamid, aufgetrennt.

Zur Auslegung und zu den Betriebsbedingungen für die zweite Kolonne wird lediglich angegeben, dass es sich um einen klassischen Kolonnentyp und klassische Betriebsbedingungen handelt. Aus den Beispielen ist zu entnehmen, dass die zweite Kolonne bei einem Druck von 400 mm Hg und einer Kopftemperatur von 143°C betrieben wird, sowie dass das gereinigte DMAc noch etwa 225 ppm Essigsäure und ebensoviel Wasser enthält, und somit den hohen Spezifikationsanforderungen, wie sie für Rein-DMAc für die Zwecke der vorliegenden Erfindung definiert sind, nicht erfüllen.

Ein spezielles, in der Praxis häufig auftretendes Problem ist der zu hohe Gehalt an Aminen im destillativ gewonnenen Rein-DMAc.

Als Amine kommen im vorliegenden Verfahren überwiegend das leichtflüchtige Dimethyl-amin vor, das einen Siedepunkt von ca. 7°C bei 1 bar absolut hat, daneben insbesondere Monomethylamin.

Um die vorstehend aufgeführte Spezifikationsanforderung für den pH-Wert im Bereich zwischen 6,5 und 7 einhalten zu können, darf die Aminkonzentration im Rein-DMAc einen Wert von etwa 5 Gew.-ppm nicht übersteigen. Amine in höherer Konzentration müssten durch Zugabe von Säuren neutralisiert werden. Mit dem Anstieg des Säuregehalts würde jedoch die elektrische Leitfähigkeit unzulässig ansteigen.

Um das Problem unzulässig hoher Aminkonzentrationen im destillativ gewonnenen Rein-DMAc zu lösen, wurde daher in der nicht vorveröffentlichten deutschen Patentanmeldung DE 103 15 214.8 eine Betriebsweise bei einem gegenüber üblichen Verfahren erhöhten Druck, und zwar einem Druck im Bereich des Normaldrucks, vörgeschlagen.

Diese Betriebsweise ist jedoch für die destillative Trennung von Stoffgemischen mit überwiegend Van der Waais-Kräften und ohne Wasserstoffbrückenbildung thermodynamisch ungünstig, da mit der Erhöhung des Druckes der Verteilungskoeffizient der leichter siedenden Komponente kleiner und somit der Trennaufwand größer wird. Dieser Effekt ist bei dem vorliegend aufzutrennenden Stoffgemisch besonders stark ausgeprägt.

Es war daher Aufgabe der Erfindung, ein Verfahren zur destillativen Gewinnung von Rein-DMAc zur Verfügung zu stellen, das die Einhaltung der geforderten Spezifikationen insbesondere für das Lösungsspinnen zur Herstellung von elastischen Polyurethan-Blockcopolymerfasem sowie zur Herstellung von Hohlfasern gewährleistet und das die Nachteile bekannter Verfahren nicht aufweist.

Darüber hinaus sollte das Verfahren die Abtrennung des Wassers aus dem Roh-DMAc in einem Reinheitsgrad, insbesondere mit einem Restgehalt an DMAc von unter 50 ppm gewährleisten, der die Wiederverwendung oder die problemlose Entsorgung desselben als Abwasser erlaubt.

Die Lösung geht aus von einem Verfahren zur destillativen Reinigung von wasserhaltigem Roh-Dimethylacetamid (Roh-DMAc), enthaltend DMAc, Leichtsieder und Schwersieder, wobei die Leichtsieder und die Schwersieder unter Erhalt von Rein-DMAc in einer oder mehreren hintereinander geschalteten Kolonnen abgetrennt werden

Die Erfindung ist dadurch gekennzeichnet, dass das Rein-DMAc als flüssiger Seitenstrom aus der einen Kolonne oder aus der letzten der mehreren hintereinander geschalteten Kolonnen abgezogen wird.

Es wurde überraschend gefunden, dass es möglich ist, auf destillativem Wege die Aminkonzentration im Rein-DMAc unterhalb von etwa 5 Gew.-ppm abzureichem und damit die geforderten Spezifikationen für Rein-DMAc einzuhalten. Dieses Ergebnis ist überraschend, da in der praktischen Durchführung von destillativen Trennverfahren im Allgemeinen die Abreicherung von Komponenten auf Restgehalte unterhalb von 10 ppm schwierig ist, insbesondere da sich schlechter durchmischte Zonen, besonders in Randbereichen von Packungen, bei der praktischen Durchführung von Destillationen nicht vollständig vermeiden lassen. Darüber hinaus finden in den Stoffgemischen, die im vorliegenden Trennverfahren eingesetzt werden, unter den Verfahrensbedingungen der Destillation sehr komplexe Reaktionen statt, die unter anderem zur Neubildung von Aminen führen.

Demgegenüber wurde unerwartet gefunden, dass die gewünschte niedrige Aminkonzentration im Wertprodukt Rein-DMAc erreicht werden kann, wenn dieses als flüssiger Seitenabzug aus der Destillation entnommen wird.

Dabei wird unter Seitenabzug wie üblich verstanden, dass in der Kolonne oberhalb der Abzugsstelle mindestens eine weitere theoretische Trennstufe vorhanden ist.

Als Leichtsieder werden vorliegend Substanzen bezeichnet, deren Siedepunkt unterhalb des Siedepunktes von DMAc (166°C bei Normaldruck) liegt und als Schwersieder Substanzen, deren Siedepunkt oberhalb des Siedepunktes von DMAc liegt.

Leichtsieder sind im vorliegenden Verfahren insbesondere Wasser, daneben Dimethylamin und Diethylamin.

Schwersieder sind insbesondere das Azeotrop von DMAc mit Essigsäure, daneben Ethanolamine, wie auch sogenannte Heavyends, d.h. hochmolekulare Zersetzungsprodukte in Form von hochviskosen Flüssigkeiten oder Feststoffen.

Als Kolonne in Abtriebsfahrweise wird eine Kolonne bezeichnet, die nur unterhalb der Zuführung des in der Kolonne aufzutrennenden Gemisches trennwirksame Einbauten aufweist. Demgegenüber weist eine Kolonne in Verstärkungsfahrweise nur oberhalb der Zuführung des in der Kolonne aufzutrennenden Gemisches trennwirksame Einbauten auf.

Als Trennwandkolonne wird in bekannter Weise eine Kolonne mit in Kolonnenlängsrichtung angeordneter Trennwand bezeichnet, die in Teilbereichen der Kolonne eine Vermischung von Flüssigkeits- und Brüdenströmen verhindert. Die Trennwand teilt den Kolonneninnenraum in einen Zuführbereich, einen Entnahmebereich, einen oberen gemeinsamen Kolonnenbereich sowie einen unteren gemeinsamen Kolonnenbereich.

Bei der vorliegenden Trennaufgabe müssen aus dem Ausgangsgemisch Roh-DMAc Leichtsieder und Schwersieder abgetrennt und das Wertprodukt, Rein-DMAc, als Mittelsieder abgezogen werden.

Für diese Trennaufgabe wird der Fachmann die im Einzelfall günstigste Kolonnenkonfiguration unter Berücksichtigung von wirtschaftlichen Überlegungen, insbesondere der Zusammensetzung des Feed-Stroms Roh-DMAc und der geforderten Spezifikation für das Rein-DMAc, aktuellen Energiekosten, usw. wählen.

Aus wirtschaftlichen Gründen, insbesondere Investitions- und Energiekosten, ist häufig die Wahl einer Trennwandkolonne für die Durchführung des erfindungsgemäßen Verfahrens besonders vorteilhaft.

Der grundsätzliche Aufbau einer Trennwandkolonne mit vertikal in Kolonnenlängsrichtung angeordneter Trennwand, die in Teilbereichen der Kolonne eine Vermischung von Flüssigkeits- und Brüdenströmen verhindert, wurde vorstehend angegeben.

Die Trennwand kann in dem Fachmann bekannter Weise ausgestaltet sein; besonders vorteilhaft ist es, dieselbe lose, insbesondere gesteckt, auszubilden.

Die im Einzelfall günstigsten trennwirksamen Einbauten wird der Fachmann nach üblichen verfahrenstechnischen Überlegungen bestimmen. Aus konstruktionstechnischen Gründen sind für die Trennwandkolonne insbesondere geordnete oder ungeordnete Packungen vorzuziehen, Böden sind weniger gut geeignet.

Die Bestimmung der im Einzelfall optimalen Trennstufenzahl im Bereich der Trennwand wird der Fachmann nach allgemeinen verfahrenstechnischen Überlegungen vornehmen und hierbei insbesondere berücksichtigen, dass, in Abhängigkeit von der vorgegebenen Spezifikation für das Wertprodukt die Leichtsiederkonzentration am unteren Ende der Trennwand einen vorgegebenen Wert nicht übersteigt und entsprechend die Schwersiederkonzentration am oberen Ende der Trennwand einen vorgegebenen Wert nicht übersteigt.

Weiterhin können für die vorliegende Trennaufgabe Kolonnenkonfigurationen aus mehreren hintereinander geschalteten Kolonnen eingesetzt werden, insbesondere eine Kolonnenkonfiguration aus einer Hauptkolonne und einer Seitenabzugskolonne, wobei das Rein-DMAc aus der Seitenabzugskolonne abgetrennt wird. Diese Kolonnenkonfiguration ist besonders vorteilhaft für die destillative Reinigung von Roh-DMAc mit relativ hohem Wassergehalt.

In Abhängigkeit von der Zusammensetzung des Roh-DMAc-Stromes, der Reinheitsanforderungen an das Rein-DMAc sowie von wirtschaftlichen Überlegungen kann es in der Kolonnenkonfiguration mit Hauptkolonne und Seitenabzugskolonne vorteilhaft sein, aus der Hauptkolonne den Seitenstrom flüssig abzuziehen und die Seitenabzugskolonne in Abtriebsfahrweise zu betreiben.

In der Kolonnenkonfiguration mit Hauptkolonne und Seitenabzugskolonne wird der Fachmann die im Einzelfall am besten geeigneten trennwirksamen Einbauten nach üblichen verfahrenstechnischen Überlegungen bestimmen und insbesondere Böden, Füllkörper oder Packungen einsetzen.

Als Einbauten eigenen sich übliche Einbauten wie handelsübliche Böden, Füllkörper oder Packungen, beispielsweise Glockenböden, Tunnelböden, Ventilböden, Siebböden, Dualflowböden und Gitterböden, Pall-Ringe^{®}, Berl^{®}-Sattelkörper, Netzdrahtringe, Raschig-Ringe^{®}, Intalox^{®}-Füllkörper und Intos^{®}, aber auch geordnete Packungen, wie beispielsweise Sulzer-Mellapak^{®}, Sulzer-Optiflow^{®}, Kühni-Rombopak^{®} und Montz-Pak^{®} sowie Gewebepackungen. Bevorzugt sind Hochleistungspackungen.

Die oben beschriebenen Packungen können gleichermaßen in der Kolonnenkonfiguration mit Trennwandkolonne eingesetzt werden.

Der Durchmesser der Kolonnen bezüglich Höhe und Positionierung von Zu- und Abläufen kann nach dem bekannten Konzept der theoretischen Trennstufen in Verbindung mit den gewählten Einbauten ermittelt werden.

Als eine theoretische Trennstufe wird in bekannter Weise diejenige Kolonneneinheit verstanden, welche ein Anreichern der leichter flüchtigen Komponente entsprechend dem thermodynamischen Gleichgewicht bewirkt, vorausgesetzt, dass ideale Durchmischung vorliegt, flüssige und gasförmige Phase im Gleichgewicht stehen und kein Mitreißen von Flüssigkeitstropfen erfolgt (vgl. Vauck, Müller: Grundoperationen chemischer Verfahrenstechnik, VCH-Verlagsgesellschaft mbH, Weinheim, 1988).

Die Zahl der theoretischen Trennstufen für den Bereich oberhalb der Zuführung des zu reinigenden Roh-DMAc-Stromes, d. h. für den oberen Bereich der Hauptkolonne oder für den Bereich oberhalb des Trennblechs in der Trennwandkolonne wird nach üblichen verfahrenstechnischen Überlegungen in Abhängigkeit von Leichtsiederanteil am Roh-DMAc und dem zulässigen Verlust an DMAc über den Kopf der ersten Kolonne festgelegt.

Die Zahl der theoretischen Trennstufen im Abtriebsteil der Hauptkolonne oder der Trennwandkolonne unterhalb des Trennblechs wird vorzugsweise im Bereich von 5 bis 30, insbesondere im Bereich von 10 bis 25, besonders bevorzugt im Bereich von 12 bis 18 festgelegt.

Bevorzugt sind die Hauptkolonne beziehungsweise die Trennwandkolonne jeweils mit einem Sumpfverdampfer und einem Kondensator am Kolonnenkopf ausgestattet.

Vorteilhaft wird die Temperatur am Kopf der Hauptkolonne oder der Trennwandkolonne in einem Bereich von 40 bis 130°C, bevorzugt in einem Bereich von 50 bis 80°C, besonders bevorzugt in einem Bereich von 50 bis 70°C, und die Temperatur im Sumpf der Hauptkolonne oder der Trennwandkolonne jeweils in einem Bereich von 90 bis 200°C, bevorzugt in einem Bereich von 110 bis 170°C, besonders bevorzugt in einem Bereich von 120 bis 140°C geregelt.

Unabhängig von der im Einzelfall gewählten Kolonnenkonfiguration kann es vorteilhaft sein, die Kolonne, aus der das Rein-DMAc abgezogen wird, das heißt insbesondere die Trennwandkolonne und die der Hauptkolonne nachgeschaltete Seitenabzugskolonne bei einem Kopfdruck < als 1 bar absolut zu betreiben, bevorzugt bei einem Kopfdruck im Bereich von 0,1 bis 0,7 bar absolut, besonders bevorzugt bei einem Kopfdruck im Bereich von 0,2 bis 0,4 bar absolut. Die Betriebsweise bei Atmosphärendruck oder unterhalb Atmosphärendruck führt insbesondere zu einer vorteilhaft kleineren Kolonnendimensionierung.

Der Schwersiederaustrag enthält neben Essigsäure auch das Wertprodukt DMAc, da Essigsäure und DMAc ein Schwersiederazeotrop bilden. Die vollständige thermische Auftrennung von Essigsäure und DMAc ist deshalb destillativ nicht möglich. Üblicherweise wird deshalb Essigsäure zunächst mit Lauge neutralisiert und das verbleibende Gemisch eingedampft. Dabei werden die Brüden, im Wesentlichen das DMAc, in die Destillation zurückgeführt und der verbleibende Rückstand entsorgt. Die Neutralisation und das anschließende Eindampfen kann sowohl kontinuierlich als auch diskontinuierlich erfolgen.

Vorteilhaft wird die destillative Reinigung kontinuierlich betrieben.

Durch das erfindungsgemäße Verfahren wird somit in überraschend einfacher Weise ein Rein-DMAc gewonnen, das die geforderten Spezifikationen bezüglich Wassergehalt, elektrischer Leitfähigkeit und insbesondere pH-Wert erfüllt.

Die Erfindung wird im Folgenden anhand einer Zeichnung und eines Ausführungsbeispiels näher erläutert.

Es zeigen im Einzelnen:
- Figur 1: ein Verfahrensschema mit Trennwandkolonne und
- Figur 2: ein Verfahrensschema mit Hauptkolonne und Seitenabzugskolonne.

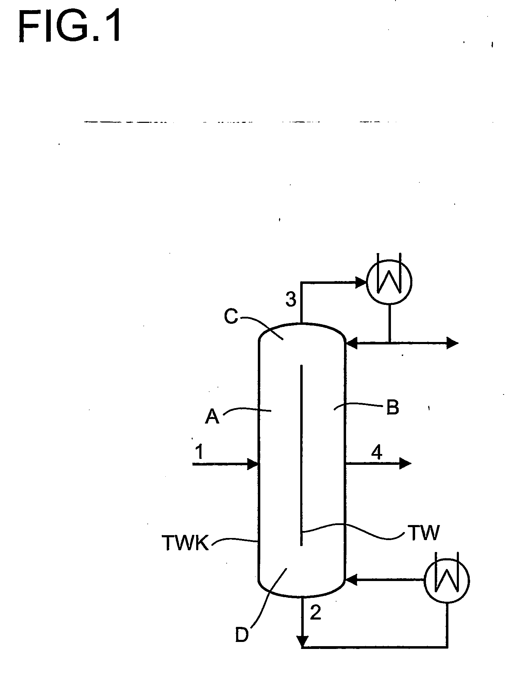

Figur 1 zeigt eine Trennwandkolonne TWK mit in Kolonnenlängsrichtung angeordneter Trennwand TW, die den Kolonneninnenraum in einen Zuführbereich A, einen Entnahmebereich B, einen oberen gemeinsamen Kolonnenbereich C sowie einen unteren gemeinsamen Kolonnenbereich D auftrennt.

Der Feed Strom 1, Roh-DMAc, wird dem Zuführbereich A der Trennwandkolonne TWK zugeführt. Aus dem unteren gemeinsamen Kolonnenbereich D wird ein Sumpfstrom 2 abgezogen, aus dem oberen gemeinsamen Kolonnenbereich C ein Kopfstrom 3 und dem Entnahmebereich B ein Seitenstrom 4, enthaltend Rein-DMAc.

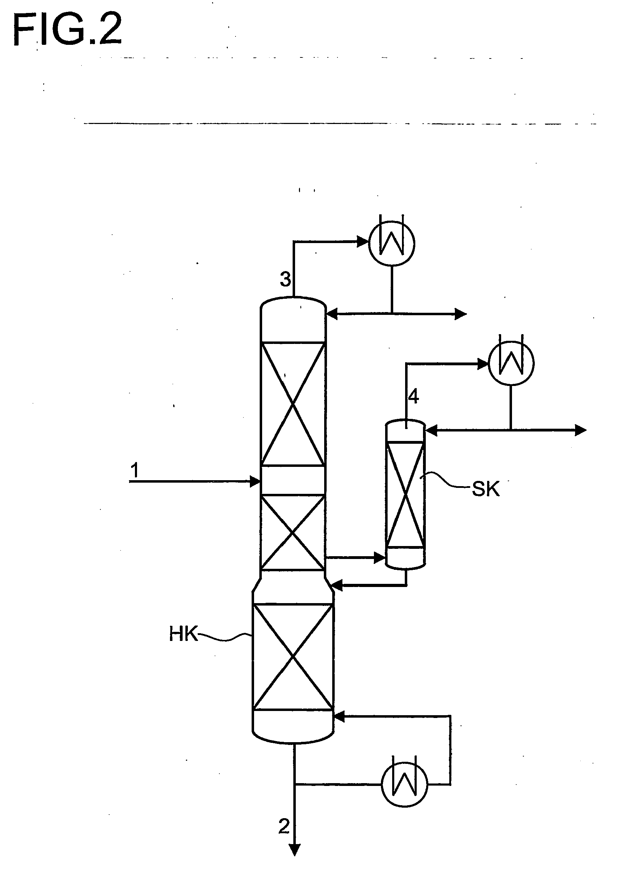

Figur 2 zeigt ein Verfahrensschema für eine Kolonnenkonfiguration mit Hauptkolonne HK und einer Seitenabzugskolonne SK. Die Ströme sind in gleicher Weise wie in Figur 1 bezeichnet, das heißt der Feed-Strom, enthaltend Roh-DMAc als Strom 1, der Sumpfstrom, in der vorliegenden Ausführungsform aus der Hauptkolonne HK, als Strom 2, der Kopfstrom, in der vorliegenden Ausführungsform aus der Hauptkolonne HK, als Strom 3 und der Rein-DMAc enthaltende Strom der in der vorliegenden Ausführungsform als Kopfstrom aus der Seitenabzugskolonne SK entnommen wird, als Strom 4.

### Ausführungsbeispiel

Einer Trennwandkolonne TWK, wie in Figur 1 schematisch dargestellt, mit 57 theoretischen Trennstufen, mit einer Trennwand TW, die sich zwischen der 18. und der 38. theoretischen Trennstufe, bei Zählung der Trennstufen von unten nach oben in der Kolonne, erstreckte, wurde ein Roh-DMAc-Strom 1 mit der folgenden Zusammensetzung zugeführt:

DMAc 99,1 Gew.-%, Dimethylamin 30 ppm, Dimethylformamid 0,2 Gew.-%, Wasser 0,3 Gew.-%, Essigsäure 1000 ppm, Ameisensäure 350 ppm, Phosphorsäure 50 ppm und Heavyends 0,2 Gew.-%.

Der Kopfdruck in der Trennwandkolonne TWK betrug ca. 250 mbar.

Auf gleicher Höhe mit dem Roh-DMAc-Strom 1, das heißt ebenfalls von der 30. theoretischen Trennstufe, wurde aus dem Entnahmebereich B der Trennwandkolonne TWK bei einer Temperatur von 125,5°C ein flüssiger Seitenstrom mit folgender Zusammensetzung abgezogen:

DMAc 99,9 Gew.-%, Dimethylformamid 720 ppm, Essigsäure 30 ppm, Wasser 80 ppm und Dimethylamin < 5 ppm. Die Zusammensetzung entsprach den Spezifikationsanforderungen für den Einsatz in der Faserherstellung.

## Patentansprüche

1. Verfahren zur destillativen Reinigung von wasserhaltigem Roh-Dimethylacetamid (Roh-DMAc), enthaltend DMAc, Leichtsieder und Schwersieder, wobei die Leichtsieder und die Schwersieder unter Erhalt von Rein-DMAc in einer Kolonne oder in mehreren hintereinander geschalteten Kolonnen abgetrennt werden, **dadurch gekennzeichnet, dass** das Rein-DMAc als flüssiger Seitenstrom (4) aus der einen Kolonne oder aus der letzten der mehreren Kolonnen abgezogen wird.

2. Verfahren nach Anspruch 1, **dadurch gekennzeichnet, dass** die eine Kolonne eine Trennwandkolonne (TWK) ist, mit in Kolonnenlängsrichtung angeordneter Trennwand (TW) die den Kolonneninnenraum in einen Zuführbereich (A), einen Entnahmebereich (B), einen oberen gemeinsamen Kolonnenbereich (C) und einen unteren gemeinsamen Kolonnenbereich (D) aufteilt, und dass das Rein-DMAc aus dem Entnahmebereich (B) der Trennwandkolonne (TWK) abgezogen wird.

3. Verfahren nach Anspruch 1, **dadurch gekennzeichnet, dass** die mehreren Kolonnen eine Hauptkolonne (HK) und eine Seitenabzugskolonne (SK) sind und dass das Rein-DMAc aus der Seitenabzugskolonne (SK) abgetrennt wird.

4. Verfahren nach einem der Ansprüche 1 bis 3, **dadurch gekennzeichnet, dass** die Kolonne, aus der das Rein-DMAc abgezogen wird, bei einem Kopfdruck kleiner als 1 bar absolut, bevorzugt bei einem Kopfdruck im Bereich von 0,1 bis 0,7 bar absolut, besonders bei einem Kopfdruck im Bereich von 0,2 bis 0,4 bar absolut, betrieben wird.

5. Verfahren nach Anspruch 2 oder 4, **dadurch gekennzeichnet, dass** die Zuführstelle für das Roh-DMAc und die Entnahmestelle für das Rein-DMAc auf gleicher Höhe in der Trennwandkolonne positioniert sind.

6. Verfahren nach einem der Ansprüche 1 bis 5, **dadurch gekennzeichnet, dass** es kontinuierlich betrieben wird.

7. Verfahren nach einem der Ansprüche 2 bis 6, **dadurch gekennzeichnet, dass** der Abtriebsteil der Hauptkolonne (HK) oder der Trennwandkolonne (TWK) 5 bis 30, bevorzugt 10 bis 25, besonders bevorzugt 12 bis 18 theoretische Trennstufen aufweist.

8. Verfahren nach einem der Ansprüche 2 bis 7, **dadurch gekennzeichnet, dass** die Trennwandkolonne (TWK) oder die Hauptkolonne (HK) jeweils einen Sumpfverdampfer und einen Kondensator am Kolonnenkopf aufweisen.

9. Verfahren nach einem der Ansprüche 2 bis 8, **dadurch gekennzeichnet, dass** die Temperatur am Kopf der Trennwandkolonne (TWK) oder der Hauptkolonne (HK) in einem Bereich von 40 bis 130°C, bevorzugt in einem Bereich von 50 bis 80°C, besonders bevorzugt in einem Bereich von 50 bis 70°C, und die Temperatur im Sumpf der Trennwandkolonne (TWK) oder der Hauptkolonne (HK) jeweils in einem Bereich von 90 bis 200°C, bevorzugt in einem Bereich von 110 bis 170°C, besonders bevorzugt in einem Bereich von 120 bis 140°C, eingestellt wird.

10. Verfahren nach einem der Ansprüche 3 bis 9, **dadurch gekennzeichnet, dass** die Hauptkolonne (HK) einen gasförmigen Seitenabzug aufweist und die Seitenabzugskolonne (SK) in Verstärkungsfahrweise betrieben wird.

## Claims

1. A process for distillatively purifying aqueous crude dimethylacetamide (crude DMAc) comprising DMAc, low boilers and high boilers, the low boilers and the high boilers being removed to obtain pure DMAc in a column or in a plurality of columns connected in series, which comprises drawing off the pure DMAc as a liquid sidestream (4) from the one column or from the last of the plurality of columns.

2. The process according to claim 1, wherein the one column is a dividing wall column (DWC), with a dividing wall (DW) arranged in the longitudinal direction of the column which divides the column interior into a feed region (A), a withdrawal region (B), an upper combined column region (C) and a lower combined column region (D), and the pure DMAc is drawn off from the withdrawal region (B) of the dividing wall column (DWC).

3. The process according to claim 1, wherein the plurality of columns are a main column (MC) and a side draw column (SC) and the pure DMAc is removed from the side draw column (SC).

4. The process according to any of claims 1 to 3, wherein the column from which the pure DMAc is drawn off is operated at a top pressure of less than 1 bar absolute, preferably at a top pressure in the range from 0.1 to 0.7 bar absolute, particularly at a top pressure in the range from 0.2 to 0.4 bar absolute.

5. The process according to claim 2 or 4, wherein the feedpoint for the crude DMAc and the withdrawal point for the pure DMAc are positioned at the same height in the dividing wall column.

6. The process according to any of claims 1 to 5, which is operated continuously.

7. The process according to any of claims 2 to 6, wherein the stripping section of the main column (MC) or of the dividing wall column (DWC) has from 5 to 30, preferably from 10 to 25, more preferably from 12 to 18, theoretical plates.

8. The process according to any of claims 2 to 7, wherein the dividing wall column (DWC) or the main column (MC) each have a bottom evaporator and a condenser at the top of the column.

9. The process according to any of claims 2 to 8, wherein the temperature at the top of the dividing wall column (DWC) or of the main column (MC) is set within a range from 40 to 130°C, preferably within a range from 50 to 80°C, more preferably within a range from 50 to 70°C, and the temperature in the bottom of the dividing wall column (DWC) or of the main column (MC) in each case within a range from 90 to 200°C, preferably within a range from 110 to 170°C, more preferably within a range from 120 to 140°C.

10. The process according to any of claims 3 to 9, wherein the main column (MC) has a gaseous side draw and the side draw column (SC) is operated in rectifying mode.

## Revendications

1. Procédé pour purifier par distillation du diméthylacétamide brut contenant de l'eau (DMAc brut), contenant du DMAc, des produits à bas point d'ébullition et des produits à haut point d'ébullition, les produits à bas point d'ébullition et les produits à haut point d'ébullition étant séparés dans une colonne ou dans plusieurs colonnes raccordées en série pour donner du DMAc pur, **caractérisé en ce que** le DMAc pur est extrait sous la forme d'un courant liquide latéral (4) à partir de la colonne unique ou à partir de la dernière colonne de la pluralité de colonnes.

2. Procédé selon la revendication 1, **caractérisé en ce que** la colonne unique est une colonne à paroi séparatrice (TWK), comprenant une paroi séparatrice (TW) disposée dans le sens longitudinal de la colonne, qui divise l'espace interne de la colonne en une zone d'alimentation (A), une zone d'extraction (B), une zone de colonne supérieure commune (C) et une zone de colonne inférieure commune (D), et **en ce que** le DMAc pur est extrait depuis la zone d'extraction (B) de la colonne à paroi séparatrice (TWK).

3. Procédé selon la revendication 1, **caractérisé en ce que** la pluralité de colonnes comprend une colonne principale (HK) et une colonne de soutirage latéral (SK), et **en ce que** le DMAc pur est séparé à partir de la colonne de soutirage latéral (SK).

4. Procédé selon l'une quelconque des revendications 1 à 3, **caractérisé en ce que** la colonne, dont on extrait le DMAc pur, fonctionne à une pression absolue de tête inférieure à 1 bar, de préférence, à une pression absolue de tête située dans la plage de 0,1 à 0,7 bar, mieux encore, à une pression absolue de tête située dans la plage de 0,2 à 0,4 bar.

5. Procédé selon la revendication 2 ou 4, **caractérisé en ce que** le site d'alimentation pour le DMAc brut et le site d'extraction pour le DMAc pur, sont positionnés à la même hauteur dans la colonne à paroi séparatrice.

6. Procédé selon l'une quelconque des revendications 1 à 5, **caractérisé en ce que** l'exploitation est réalisée en mode continu.

7. Procédé selon l'une quelconque des revendications 2 à 6, **caractérisé en ce que** la partie de rectification de la colonne principale (HK) ou de la colonne à paroi séparatrice (TWK) présente 5 à 30, de préférence, 10 à 25 en particulier 12 à 18 étages de séparation théoriques.

8. Procédé selon l'une quelconque des revendications 2 à 7, **caractérisé en ce que** la colonne à paroi séparatrice (TWK) ou la colonne principale (HK) présentent, respectivement, un évaporateur de bas de colonne et un condenseur en tête de colonne.

9. Procédé selon l'une quelconque des revendications 2 à 8, **caractérisé en ce que** la température de tête de la colonne à paroi séparatrice (TWK) ou de la colonne principale (HK) est réglée dans une plage de 40 à 130 °C, de préférence dans une plage de 50 à 80 °C, mieux encore dans une plage de 50 à 70 °C, et la température de bas de la colonne à paroi séparatrice (TWK) ou de la colonne principale (HK), respectivement, est réglée dans une plage de 90 à 200 °C, de préférence dans une plage de 110 à 170 °C, mieux encore dans une plage de 120 à 140 °C.

10. Procédé selon l'une quelconque des revendications 3 à 9, **caractérisé en ce que** la colonne principale (HK) présente un soutirage latéral de gaz, et **en ce que** la colonne de soutirage latéral (SK) fonctionne en mode renforcé.
